# EUROPÄISCHE PATENTSCHRIFT

(11) **EP 0 945 533 B1**
(45) Veröffentlichungstag und Bekanntmachung des Hinweises auf die Patenterteilung: **11.06.2003**
(21) Anmeldenummer: 99105358.8
(22) Anmeldetag: 16.03.1999
(51) Int. Cl.: D01H 13/22

(54) **Vorrichtung zum Messen von Eigenschaften eines längsbewegten Prüfguts**
Apparatus for measuring the properties of a material sample in longitudinal movement
Appareil pour mesurer les propriétés d'un échantillon de matériau en mouvement longitudinal

(30) Priorität: 25.03.1998 CH 69898
(43) Veröffentlichungstag der Anmeldung: 29.09.1999
(73) Patentinhaber: Uster Technologies AG, 8610 Uster (CH)
(72) Erfinder: Brunner, Felix, 8620 Wetzikon (CH); Hiltebrand, Bernhard, 8610 Uster (CH); Schilling, Peter, 8854 Siebnen (CH); Keller, Beat, 8600 Dübendorf (CH); Wepfer, Hanspeter, 8476 Unterstammheim (CH); Emch, Beat, 8193 Eglisau (CH); Joss, Rolf, 8810 Horgen (CH)

(56) Entgegenhaltungen:
- EP-A- 0 679 862
- EP-A- 0 821 089
- WO-A-97/36032
- DE-A- 3 635 267

## Beschreibung

Die Erfindung betrifft eine Vorrichtung zum Messen von Eigenschaften eines längsbewegten Prüfguts in einer Messzelle.

Solche Vorrichtungen (siehe DE-A-3 635 267) weisen, insbesondere dann, wenn sie zum Messen von Eigenschaften von Gam vorgesehen und an einer Spinn- oder Spulmaschine angeordnet sind, drei durch Leitungen verbundene Teile auf, nämlich den Messkopf mit der Messzelle, die Auswerteeinheit und das Steuergerät Dabei sind an ein Steuergerät eine oder mehrere Auswerteeinheiten und an jede Auswerteeinheit ein- oder mehrere Messköpfe angeschlossen. Die Messköpfe geben unverarbeitete, analoge oder auch analoge und digitale Signale an die Auswerteeinheit ab. Die Auswerteeinheit gibt aber auch Signale an den Messkopf ab. Die Auswerteeinheit wertet die Signale aus, beispielsweise indem sie diese mit Schwellwerten vergleicht und gibt verarbeitete und eventuell auch digitalisierte Signale an das Steuergerät ab. Üblicherweise ist pro Spinnmaschine oder pro Spulmaschine mit einem Steuergerät zu rechnen.

Solche bekannte Vorrichtungen benötigen viel Raum. Um in der Spinn- oder Spulmaschine am Gam möglichst wenig Raum zu beanspruchen, ist der Messkopf möglichst einfach ausgebildet So kann er nur eine einzige Funktion, nämlich das Messen, ausüben. Andere Funktionen werden deshalb in der Auswerteeinheit ausgeübt. Das bedeutet, dass eine solche bekannte Vorrichtung viele Teile aufweist, die über viele Leitungen und Steckverbindungen miteinander verbunden sind und dementsprechend teuer in der Anschaffung ist So ist auch der Anbau an die Spinn- und Spulmaschine aufwendig und komplex. Da jeder Messkopf auch spezifisch und im Hinblick auf die Maschine ausgebildet ist, an die er angebaut werden soll, heisst dies auch, dass für jede Bauart einer Spinn- oder Spulmaschine ein spezieller Messkopf und eine eigene Auswerteeinheit hergestellt wird.

Die Erfindung, wie sie in den Patentansprüchen gekennzeichnet ist, löst deshalb die Aufgabe, eine Vorrichtung zu schaffen, die einfacher an die Bedürfnisse eines Kunden und die speziellen Verhältnisse an einer Produktionsmaschine für längsbewegtes Prüfgut anzupassen ist.

Dies wird mit einer Vorrichtung erreicht, bei der eine Messzelle für das längsbewegte Prüfgut direkt an einen zugeordneten und nur für diese Messzelle vorgesehenen Prozessor angeschlossen ist. Die Messzelle und der Prozessor sind zusammen unlösbar verbunden und auf einem ersten Träger angeordnet oder in einem eigenen Gehäuse zusammengefasst. Dabei gibt es nur einen Ausgang für eine Leitung zur Übertragung von digitalisierten und bereits einer ersten Auswertung unterzogenen Signalen. Eine solche Vorrichtung kann mehrere Messzellen für verschiedene Aufgaben aufweisen, die zusammen wiederum auf einem weiteren Träger oder in einem weiteren Gehäuse angeordnet sind. Als verschiedene Aufgaben sind beispielsweise verschiedenartige Messungen zu verstehen, beispielsweise optische oder kapazitive Messungen des Querschnitts oder der Masse des Prüfguts, Fremdstoffmessungen, Messungen der Haarigkeit an einem Garn usw. Der Prozessor ist vorzugsweise als sogenannter ASIC-Baustein, also als kundenspezifischer integrierter Bauteil aufgebaut Er ist immer gleich aufgebaut und kann dieselben Funktionen ausüben, unabhängig davon ob er an eine Messzelle für optische Messung, eine solche für kapazitive Messung usw. angeschlossen ist. Insbesondere dient er der Verstärkung und Umwandlung von Signalen. Deshalb weist der ASIC-Baustein mindestens einen Analog-Digital-Wandler auf. So weist er oft mehrere Eingänge für analoge Signale und einen Ausgang für digitale Signale auf. Der weitere Träger weist neben der oder den ersten Trägem oder Gehäusen mit den Messzellen einen Prozessor, insbesondere einen digital arbeitenden Signalprozessor (DSP) auf, der mehrere Eingänge für Anschlüsse an mehrere ASIC-Bausteine aufweist. Die ersten Träger mit den Messzellen sind über lösbare Verbindungen z.B. Stecker mit dem weiteren Träger verbunden.

Die durch die erfindungsgemässe Vorrichtung erreichten Vorteile sind insbesondere darin zu sehen, dass eine separate, in einigem Abstand zum Messkopf vorgesehene Auswerteeinheit entfallen kann. Damit sind wesentlich weniger Verbindungen zwischen Messköpfen, Auswerteeinheiten und Steuergeräten an einer Spinn- oder Spulmaschine vorhanden, was, weil damit auch viele Steckverbindungen und Leiterplatten entfallen, die Betriebssicherheit erhöht. Da die Vorrichtung nur mehr wenige standardisierteTeile aufweist, wird die Herstellung und Lagerhaltung dieser Teile beim Hersteller ebenfalls vereinfacht. Da von jedem Teil grössere Stückzahlen erreicht werden, können diese auch genauer und mit grösserem Aufwand geprüft werden, weil dazu eigene Prüfvorrichtungen lohnend sind. Da die "Intelligenz" somit in die Messköpfe ausgelagert und dezentralisiert ist, ist auch nur noch ein Kabel vom Messkopf zu der Produktionsstelle vorzusehen und dieses Kabel transportiert digitale Signale, was die Sicherheit des Systems gegen Störungen erhöht. Die Software ist bereits im Messkopf gespeichert und muss nicht mehr dort bei Bedarf geladen werden.

Im folgenden wird die Erfindung anhand eines Beispiels und mit Bezug auf die beiliegenden Figuren näher erläutert. Es zeigen:
Figur 1 eine Darstellung der erfindungsgemässen Vorrichtung, wobei die einzelnen Teile auseinandergezogen sind, und
Figur 2 eine perspektivische Darstellung der Vorrichtung von aussen.

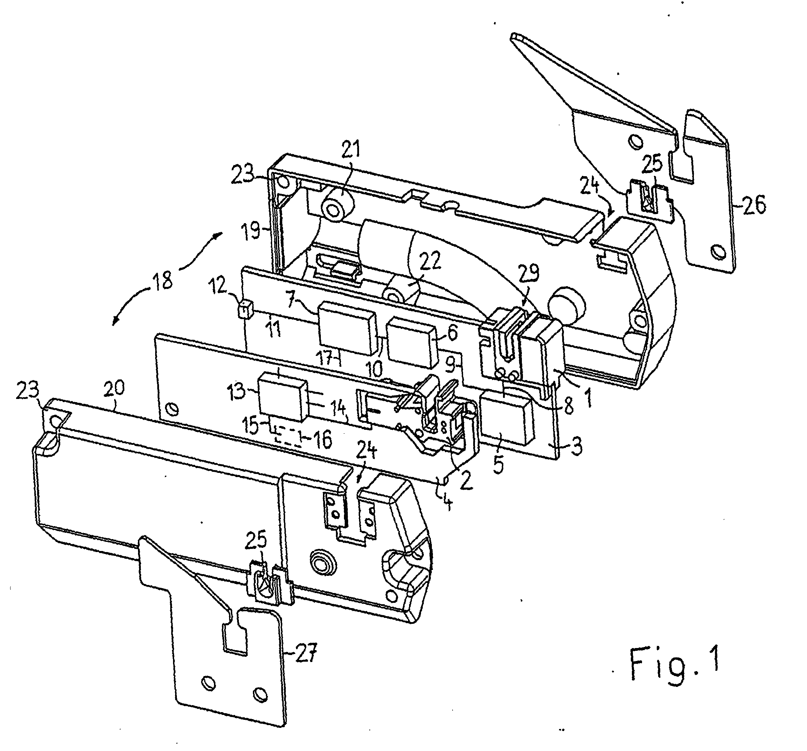

Fig. 1 zeigt eine Vorrichtung zum Messen von Eigenschaften an einem längsbewegten Prüfgut mit einer ersten Messzelle 1 und einer zweiten Messzelle 2. Beispielsweise kann die erste Messzelle 1 ein optisch oder kapazitiv arbeitender Sensor sein, wie er bereits in bekannten Gamreinigem im Einsatz ist und den Querschnitt oder die Masse des Prüfguts misst. Die zweite Messzelle 2 kann beispielsweise als Sensor ausgebildet sein, der Fremdstoffe im Prüfgut detektiert, oder der andere Eigenschaften des Prüfguts, wie beispielsweise die Haarigkeit, die Färbung, die Verdrehung usw. misst. Dabei ist die Messzelle 1 und die Messzelle 2 je auf einem ersten Träger 3 bzw. 4 befestigt. Der erste Träger 3, 4 ist beispielsweise als Leiterplatte ausgebildet. Auf dem ersten Träger 3 sind weitere Bauteile befestigt, nämlich je ein Prozessor 5 und 6, sowie ein Rechner 7. Der Prozessor 5 ist als sogenannter ASIC ausgebildet und verstärkt die von der Messzelle 1 ausgegebenen Signale. Dieser Prozessor 5 ist auf die Messzelle 1 abgestimmt und verarbeitet analoge Signale. Der Prozessor 6 ist ebenfalls als ASIC (kundenspezifische integrierte Schaltung) ausgebildet, aber für gemischte Signale ausgelegt. D. h. er kann analoge und digitale Signale verarbeiten. Der Rechner 7 ist als digitaler Signalprozessor (sog. DSP) ausgebildet. Damit ist der Rechner 7 über die beiden Prozessoren 5 und 6 und über Leitungen 8, 9 und 10 mit der Messzelle 1 unlösbar verbunden, denn alle diese und auch weitere Elemente sind gemäss den üblichen Methoden auf den als Leiterplatte ausgebildeten ersten Träger 3 fest aufgebracht. Der Rechner 7 ist weiter über eine Leitung 11 mit einer Steckverbindung 12 verbunden. Die Leitungen 8 und 9 übertragen analoge Signale, während die Leitungen 10 und 11, welche Ausgänge für den Prozessor 6 und den Rechner 7 bilden, digitale Signale übertragen.

Der erste Träger 4 mit der Messzelle 2 weist ebenfalls einen Prozessor 13 auf, der über eine Leitung 14 mit der Messzelle 2 verbunden ist Der Prozessor 13 ist gleich aufgebaut wie der Prozessor 6. Insbesondere verwendet man für die Prozessoren 6 und 13 identische Bauteile. Auch dieser erste Träger 4 ist vorzugsweise als Leiterplatte ausgebildet. Der Prozessor 13 weist zudem einen weiteren Ausgang 15 auf, der in eine Steckverbindung 16 mündet, die zwischen den beiden ersten Trägern 3 und 4 vorgesehen ist, und in die auf dem ersten Träger 3 eine Leitung 17 mündet, die an den Rechner 7 angeschlossen ist.

Neben den hier gezeigten beiden ersten Trägern 3 und 4 ist ein weiterer Träger 18 vorgesehen, der hier aus zwei Teilen 19, 20 besteht und ein Gehäuse für die beiden ersten Träger 3, 4 mit den Messzellen 1, 2 bildet. Die beiden ersten Träger 3, 4 sind über Schraubverbindungen an den beiden Teilen 19, 20 festgemacht. Hier erkennt man deshalb Teile der Schraubverbindungen 21, 22. Die beiden Teile 19, 20 sind ebenfalls über Schraubverbindungen 22, 23 aneinander befestigt. Der weitere Träger 18 ist seinerseits über Verbindungen beispielsweise auf einer Spinn- oder Spulmaschine befestigt Der weitere Träger weist insbesondere auch eine Öffnung 24 für das Prüfgut auf, und in diese Öffnung kann wiederum ein Führungselement 25 eingesetzt sein, das hier abstehend gezeigt ist. Weitere Führungselemente 26, 27 sind zum erleichterten Einlegen des Prüfguts in die Öffnung 24 vorgesehen und ebenfalls am weiteren Träger 18 befestigt.

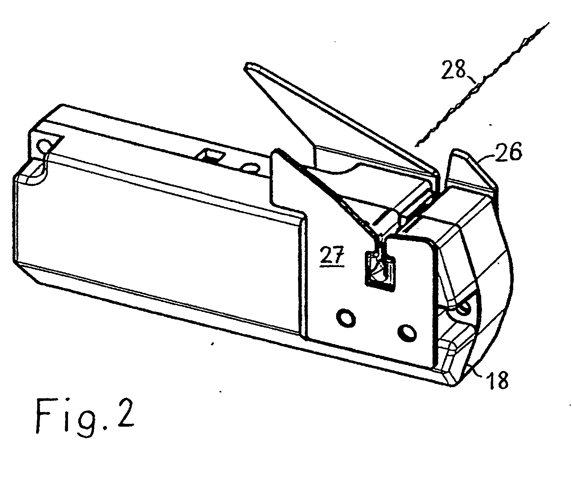

Fig. 2 zeigt hier die zusammengesetzte Vorrichtung mit den Führungselementen 26, 27, der Öffnung 24 für ein Prüfgut 28 und dem weiteren Teil 18.

Die Wirkungsweise der erfindungsgemässen Vorrichtung ist wie folgt Wird bei zusammengesetzter Vorrichtung ein Prüfgut in die Öffnung 29 in der Messzelle 1 eingeführt und darin in an sich bekannter Weise in seiner Längsrichtung bewegt, so werden in der Messzelle 1 analoge Signale erzeugt, die über die Leitung 8 in den Prozessor 5 gelangen. Dort werden sie verarbeitet und über die Leitung 9 dem Prozessor 6 zugeleitet, der diese Signale in digitale Signale wandelt Die Verarbeitung kann beispielsweise darin bestehen, dass der Prozessor 5 der Messzelle ein Trägersignal zuführt, das durch das Prüfgut moduliert wird, wobei das modulierte Signal im Prozessor 5 demoduliert und verstärkt wird. Im Prozessor 6 werden die Signale des Prüfguts digitalisiert und gefiltert Dabei kann der Prozessor 6 aber auch Steuersignale an den Prozessor 5 abgeben, z.B. um die Wirkung von Verschmutzungen in der Öffnung auszugleichen oder zum Verschwinden zu bringen. Über die Leitung 10 werden die digitalisierten Signale an den Rechner 7 ausgegeben, der diese beispielsweise mit Schwellwerten vergleicht und Verarbeitungsschritte wie z.B. die Anwendung von Fehleralgorithmen und weitere Auswertungen durchführt. Über die Leitung 11 und den Ausgang oder die Steckverbindung 12 gelangen die verarbeiteten und ausgewerteten Signale beispielsweise in die zentrale Steuerung der Maschine, auf der diese Vorrichtung befestigt ist. Es ist möglich, den Prozessor 5 auch wegzulassen und die Messzelle 1 direkt mit dem Prozessor 6 zu verbinden. Doch dies richtet sich nach der Art der verwendeten Messzelle 1 und den Signalen, die diese abgibt. Arbeitet die Messzelle 1 nach einem optischen Prinzip so trifft dies zu. Arbeitet sie nach einem kapazitiven Messprinizip, so ist der Prozessor 5 sehr erwünscht.

Neben der Messzelle 1 ist auch die Messzelle 2 vorhanden, die das Prüfgut ebenfalls misst oder abtastet Diese gibt ihr Signal über die Leitung 14 an den Prozessor 13 ab, der das Signal verstärkt und in ein digitales Signal umwandelt. Dann kann es über den Ausgang 15, die Steckverbindung 16 und die Leitung 17 in den Rechner 7 gelangen, wo es ebenfalls verarbeitet und ausgewertet wird. Der Rechner 7 hat hier auch die Möglichkeit die Signale aus beiden Messzellen 1 und 2 gemeinsam auszuwerten und aus beiden Signalen durch Kombination errechnete Werte über die Leitung 11 auszugeben.

Die Messzellen sind auf dem jeweiligen ersten Träger unlösbar mit dem Prozessor verbunden und bilden zusammen ein Modul. Ein Gehäuse oder ein weiterer Träger kann mehrere erste Träger oder Module aufnehmen. Alle diese Module geben ihr bereits digitalisiertes Signal an den einzigen Rechner 7 ab, der auf dem Hauptmodul, hier der erste Träger 3, vorhanden ist. Allerdings wäre es auch denkbar auf mehreren Modulen oder gar auf jedem Modul einen Rechner vorzusehen. Vorzugsweise ergibt sich aber für die gesamte Vorrichtung oder den Messkopf ein einziger Ausgang 12 für ausgewertete Signale, die einer Maschinensteuerung zugeführt werden können. Die verwendeten Prozessoren 5, 6, 13 haben alle den gleichen Aufbau und können alle die gleichen Funktionen ausüben. Es werden aber nicht immer alle Funktionen benützt. Beispielsweise üben die Prozessoren 5 und 6 verschiedene Verarbeitungen aus, die wiederum nicht mit der Verarbeitung im Prozessor 13 genau übereinstimmt. So haben die Prozessoren 5, 6 und 13 mehrere Eingänge 30 für analoge Signale, die, je nach vorgesehener Verarbeitung belegt werden, aber nur einen Ausgang 10, 15 für digitale Signale. So ist jeder Messzelle ein gleicher für viele Funktionen ausgelegter Baustein 5, 6, 13 zugeordnet und untrennbar mit ihr verbunden.

## Patentansprüche

1. Vorrichtung zum Messen von Eigenschaften eines längsbewegten Prüfguts in einer Messzelle, **dadurch gekennzeichnet, dass** die Messzelle (1) an einen nur der Messzelle zugeordneten Prozessor (6) angeschlossen ist und zusammen mit dem Prozessor, einer Verbindung (12) zu einer zentralen Steuerung für mehrere Vorrichtungen und Leitungen (9, 10, 11) zur Übertragung von Signalen der Messzelle, auf einem Träger (3) befestigt ist und der Träger an einer Produktionsstelle einer Spinn- oder Spulmaschine angeordnet ist und eine Verbindung (17) zu einem weiteren Träger (4) für eine weitere Messzelle (2) aufweist.

2. Vorrichtung nach Anspruch 1, **dadurch gekennzeichnet, dass** die Messzelle und der Prozessor zusammen auf einem ersten Träger (3, 4) angeordnet sind und der Prozessor (6, 13) einen Ausgang (10, 15) für digitale Signale aufweist.

3. Vorrichtung nach Anspruch 2, **dadurch gekennzeichnet, dass** ein weiterer Träger (18) vorgesehen ist, auf dem mindestens ein erster Träger (3) mit einer Messzelle (1) befestigt ist.

4. Vorrichtung nach Anspruch 3, **dadurch gekennzeichnet, dass** auf dem weiteren Träger (18) erste Träger (3, 4) mit Messzellen (1, 2) angeordnet sind, die verschiedenartige Messungen durchführen können.

5. Vorrichtung nach Anspruch 4, **dadurch gekennzeichnet, dass** als Messungen je eine aus einer Gruppe, bestehend aus optischer Messung des Querschnittes, kapazitiver Messung der Masse und Messung von Fremdstoffen, vorgesehen sind.

6. Vorrichtung nach Anspruch 1, **dadurch gekennzeichnet, dass** jeder Messzelle (1, 2) als Prozessor (6, 13) ein sogenannter ASIC-Baustein zugeordnet ist, der unabhängig von der Messzelle und deren Messung den gleichen Aufbau aufweist und die gleichen Funktionen ausüben kann.

7. Vorrichtung nach Anspruch 6, **dadurch gekennzeichnet, dass** der ASIC-Baustein Eingänge (30) für analoge Signale und einen Ausgang (10. 15) für digitale Signale aufweist.

8. Vorrichtung nach Anspruch 3, **dadurch gekennzeichnet, dass** der erste Träger (3) einen als digital arbeitenden Signalprozessor ausgebildeten Rechner (7) aufweist, der einen einzigen Ausgang (11) für Signale aus Messzellen (1, 2) auf dem weiteren Träger aufweist.

9. Vorrichtung nach Anspruch 6, **dadurch gekennzeichnet, dass** die Messzelle und der ASIC-Baustein unlösbar miteinander verbunden sind.

## Claims

1. Device for measuring properties of a longitudinally moved test material in a measuring cell, **characterised in that** the measuring cell (1) is connected to a processor (6) associated exclusively with the measuring cell and the measuring cell is fastened on a first carrier (3) together with the processor, a connector (12) to a central control system for a plurality of devices of this type and lines (9, 10, 11) used to transmit signals from the measuring cell and **in that** the carrier is fastened to a production point of a spinning or spooling frame and has a line (17) to a further carrier (4) with a further measuring cell (2).

2. Device according to claim 1, **characterized in that** the measuring cell and the processor are disposed together on a first carrier (3, 4) and the processor (6, 13) has one output (10, 15) for digital signals.

3. Device according to claim 2, **characterized in that** a further carrier (18) is provided, to which at least one first carrier (3) with a measuring cell (1) is fastened.

4. Device according to claim 3, **characterized in that** disposed on the further carrier (18) are first carriers (3, 4) with measuring cells (1, 2), which may effect various types of measurement.

5. Device according to claim 4, **characterized in that**, as measurements, one each from a group comprising optical measurement of the cross section, capacitive measurement of the mass and measurement of impurities are provided.

6. Device according to claim 1, **characterized in that** associated with each measuring cell (1, 2) as a processor (6, 13) is a so-called ASIC module which, irrespective of the measuring cell and its measuring operation, is of the same construction and may perform the same functions.

7. Device according to claim 6, **characterized in that** the ASIC module has inputs (30) for analog signals and one output (10, 15) for digital signals.

8. Device according to claim 3, **characterized in that** the first carrier (3) comprises a computer (7) in the form of a digital signal processor, which has a single output (11) for signals from measuring cells (1, 2) on the further carrier.

9. Device according to claim 6, **characterized in that** the measuring cell and the ASIC module are permanently connected to one another.

## Revendications

1. Dispositif de mesure de propriétés d'un produit à contrôler se déplaçant dans le sens longitudinal dans une cellule de mesure, **caractérisé en ce que** la cellule de mesure (1) est relié à un processeur (6) attribué seulement à la cellule de mesure et est fixée avec le processeur, un raccordement (12) avec un contrôle central pour plusieurs dispositifs et des conducteurs (9, 10, 11) pour la transmission de signaux de la cellule de mesure, sur un support (3) et **en ce que** le support est disposé sur un poste de production d'un métier à filer ou d'un bobinoir et **en ce qu'**il comporte un raccordement (17) avec un support (4) supplémentaire pour une cellule de mesure (2) supplémentaire.

2. Dispositif selon la revendication 1, **caractérisé en ce que** la cellule de mesure et le processeur sont disposés ensemble sur un premier support (3, 4) et **en ce que** le processeur (6, 13) comporte une sortie (10, 15) pour signaux numériques.

3. Dispositif selon la revendication 2, **caractérisé en ce qu'**un support (18) supplémentaire est prévu, sur lequel au moins un premier support (3) est fixé avec une cellule de mesure (1).

4. Dispositif selon la revendication 3, **caractérisé en ce que**, sur le support supplémentaire (18) sont disposés des premiers supports (3, 4) avec des cellules de mesure (1, 2), pouvant réaliser divers types de mesures.

5. Dispositif selon la revendication 4, **caractérisé en ce que**, en tant que mesures de chaque groupe, une mesure optique de la section, de la mesure capacitive de la masse et une mesure d'impuretés soient prévues.

6. Dispositif selon la revendication 1, **caractérisé en ce qu'**à chaque cellule de mesure (1, 2) soit attribué un processeur (6, 13) configuré comme un ASIC, qui présente la même structure et réalise les mêmes fonctions quelle que soit la cellule de mesure et ses mesures.

7. Dispositif selon la revendication 6, **caractérisé en ce que** le composant ASIC comporte des entrées (30) pour signaux analogiques et une sortie (10, 15) pour signaux numériques.

8. Dispositif selon la revendication 3, **caractérisé en ce que** le premier support (3) comporte un calculateur (7) configuré comme un processeur de signaux fonctionnant en numérique, qui comporte une seule sortie (11) pour les signaux provenant des cellules de mesure (1, 2) sur le support supplémentaire.

9. Dispositif selon la revendication 6, **caractérisé en ce que** la cellule de mesure et le composant ASIC sont reliés entre eux de manière fixe.
